# EUROPEAN PATENT APPLICATION

(11) **EP 4 260 902 A2**
(43) Date of publication of application: **18.10.2023**
(21) Application number: 23167393.0
(22) Date of filing: 11.04.2023
(51) Int. Cl.: A61N 5/10

(54) **PARTICLE BEAM MODULATION SYSTEMS AND METHODS**

(30) Priority: 15.04.2022 US 202217722146
(71) Applicant: Varian Medical Systems, Inc., Palo Alto, CA 94304 (US)
(72) Inventor: KRIEGER, Miriam, 06114 Halle (Saale) (DE); FOLKERTS, Michael, 92626 Costa Mesa, CA (US); BRÄUER, Martin, 90427 Nürnberg (DE)
(74) Representative: Foster, Mark Charles

(57) **Abstract**

Presented systems (100) and methods (1100) enable efficient and effective radiation treatment planning and treatment, including accurate and convenient transmission of the radiation (105) towards a tissue target (108). In one embodiment, a workflow for applying an irradiation scheme involving multiple steps is utilized to provide modulation of a particle beam (105). The workflow can include development (1210) of a treatment plan, generating configuration information for a modulation component based on the treatment plan, fabricating (1220) the modulation component, and using the modulation component in a system that delivers (1240) a radiation dose in accordance with the treatment plan.

## Description

### BACKGROUND

Radiation therapy is utilized in various medical treatments. Radiation beams are utilized in a number of different applications and accurately applying an appropriate amount of radiation is very important. Radiation therapy usually involves directing a beam of high energy proton, photon, ion, or electron radiation ("therapeutic radiation") into a tissue target or tissue target volume (e.g., a tissue volume that includes a tumor, lesion, etc.). The radiation beams are typically used to stop the growth or spread of the targeted tissue cells by killing them or degrading their cell division ability. While radiation therapy is generally considered beneficial, there are a number of potential side effects. The side effects can include unintended damage to DNA of healthy tissue cells. The effectiveness of radiation therapy is primarily a function of the dose or amount of ionizing radiation that is applied to an intended tissue target (e.g., tumor, cancerous cells, etc.) while avoiding impacts to healthy cells.

Various treatment approaches have characteristics that can potentially offer significant benefits. It was recently discovered that delivering a therapeutic dose at ultra-high dose rates (e.g., >40 Gy/s, etc.), referred to as FLASH dose rate delivery, reduces the radiation sensitivity of healthy tissue, but not of tumors. Delivering the same dose, but at ultra-high dose rates can increase the therapeutic ratio over conventional treatment delivery. Proton and electron radiation approaches can generally provide higher dose rates. While the potential benefits of using proton therapy radiation are significant, the realization of this objective has traditionally been very challenging in practice (e.g., not practical, not possible, etc.).

In particular, utilizing FLASH approaches with raster grid delivery patterns has been very difficult or impractical to implement. Scan delivery patterns typically attempt to provide a conformal (homogenous) dose, however achieving a FLASH dose rate (e.g., FLASH dose rates of 20-40 grays (Gy) delivered in less than one second, and as much as 120 or more Gy per second) with conventional scan delivery patterns is problematic. For example, reaching typical high FLASH dose rates traditionally involves a tradeoff between time and particle beam energy or current. Low energy/currents typically cannot adequately or practically provide the FLASH dose rate. High energy/currents can provide FLASH dose rates, but traditional systems typically require the energy to be changed as the particle beam traverses through a scan pattern. Conventional systems also typically require the particle beam traversal of the scan pattern to pause or stop each time the energy/current of a particle beam is changed/altered. Constantly pausing to change energies tends to slow the overall operation down which is somewhat problematic given the basic idea of FLASH treatments is to operate in a relatively fast or high dose rate regime. These repeated stops and pauses for the numerous required energy changes makes the overall time impractical and prohibitive. Traditional systems usually take 0.25s to 5s to do an energy change. With typical fields consisting of 40 Iso-Energy-Slices (IES) and 5000 points, technical experience shows that the time to change beam energy effectively prevents conventional irradiations systems from being used for FLASH therapy.

### SUMMARY

In accordance with a first aspect of the invention, there is provided a system as defined by claim 1.

In accordance with a second aspect of the invention, there is provided a method as defined by claim 12.

Optional features are defined by the dependent claims.

Presented systems and methods enable efficient and effective radiation planning and treatment, including accurate and convenient transmission of the radiation towards a tissue target. In one embodiment, a workflow for applying an irradiation scheme involving multiple steps is utilized to provide modulation of a particle beam. The workflow includes development of a treatment plan, generating configuration information for a modulation component based on the treatment plan, fabricating the modulation component, and using the modulation component in a system that delivers a radiation dose in accordance with the treatment plan. The workflow includes fabrication/construction of a modulation component according to multiple approaches or schemes, quality control, and the fast application of a particle beam with one accelerator energy. A modulation scanning component may control movement of the particle beam in a scan pattern and modulation of the particle beam resulting in a modulated particle treatment beam. The modulation scanning component can include a scan component or sub-component that directs the particle beam movement in the scan pattern and a modulation component or subcomponent that performs the modulation. In one embodiment, a modulation component includes modulation pin cells that provide modulation of a particle beam. Modulation features/characteristics of a modulation pin cell include: a) a depth shifting part/portion and b) a distal widening part/portion. An individual modulation component pin cell corresponds to a scan spot/cell position within a scan pattern. A depth shifting part/portion and a distal widening part/portion are assigned to the scan spot/cell position. A particle beam irradiation system is utilized for delivery and application of radiation to a patient.

In one embodiment, a system comprises:a particle generation component that generates a particle beam; a modulation scanning component that controls movement of the particle beam in a scan pattern and modulation of the particle beam resulting in a modulated particle treatment beam; and a treatment and configuration control component that directs configuration of the modulation scanning component and directs delivery of a treatment particle beam. The configuration of the modulation scanning component and delivery of the treatment beam are based upon a treatment plan, wherein the particle generation component generates a particle beam at a same energy level for a first portion of the scan pattern and a second portion of the scan pattern, and the modulation scanning component modulation of the treatment particle beam is different for the first portion of the scan pattern than the second portion of the scan pattern. The range of the particle treatment beam is different for the first portion of the scan pattern and the second portion of the scan pattern. The modulation scanning component adjustment of a treatment beam and radiation can include shifting the largest deposition depth to lower depths. A homogeneous range-shifting component may uniformly shift all spots to a more proximal depth. This component may be part of the modulator pins, i.e., every pin consists of a certain length that completely fills out the cell laterally. Or it may be a separate component, such that every pin only consists of the actual range modulating part and an additional block of range-shifting material is required. The modulation scanning component adjustment of a treatment beam and radiation include generating a determined dose profile from a largest penetration depth to a smallest penetration depth. The determined dose profile is homogenous from the largest penetration depth to the smallest penetration depth. The modulation scanning component adjustment of the treatment particle beam applies fields with many Iso-Energy-Slices (IES) and the particle beam is at the same energy level for the first portion of the scan pattern and the second portion of the scan pattern. Treatment fields are irradiated as single IES fields by the treatment particle beam. The modulation scanning component can include homogenous and field individual modulation components that allow a conformal irradiation using the particle beam at the same energy level.

In one embodiment, a method relates to a treatment plan creation process. A modulation component configuration process is performed where a modulation component is configured based on the treatment plan. Next, the method performs a quality assurance process, including a quality assurance process on the modulation component and also performs a treatment process in accordance with the treatment plan. The treatment plan creation process can include planning and performing a CT scan of a patient.

In one embodiment a system comprises: a particle generation component that generates a particle beam; a modulation scanning component that controls movement of the particle beam in a scan pattern and modulation of the particle beam resulting in a modulated particle treatment beam; and a treatment and configuration control component that directs configuration of the modulation scanning component and directs delivery of a treatment particle beam, wherein the configuration of the modulation scanning component and delivery of the treatment beam are based upon a treatment plan, wherein the particle generation component generates a particle beam at a same energy level for a first portion of the scan pattern and a second portion of the scan pattern, wherein the modulation scanning component is partitioned into a plurality of pin cells in which a first one of the plurality of pin cells and a second one of the plurality of pin cells have different configurations that result in different modulation of the particle beam.

This summary is provided to introduce a selection of concepts that are further described below in the detailed description that follows. This summary is not intended to identify key features or essential features of the claimed subject matter, nor is it intended to be used to limit the scope of the claimed subject matter.

### BRIEF DESCRIPTION OF DRAWINGS

The accompanying drawings, which are incorporated in and form a part of this specification and in which like numerals depict like elements, illustrate embodiments of the present disclosure and, together with the detailed description, serve to explain the principles of the disclosure. The drawings are not intended to limit the present invention to the particular implementations illustrated therein. The drawings are not to scale unless otherwise specifically indicated.
Figure 1 is a block diagram of an exemplary system in accordance with one embodiment.
Figure 2 is a block diagram of an exemplary system in accordance with one embodiment.
Figure 3 illustrates a block diagram of an exemplary radiation treatment system in accordance with one embodiment.
Figure 4 is a block diagram of an exemplary grid pattern in accordance with one embodiment.
Figure 5 is a block diagram of an exemplary modulation component in accordance with one embodiment.
Figure 6 is a three-dimensional (3D) block diagram of an exemplary modulation component in accordance with one embodiment.
Figure 7 includes three-dimensional (3D) block diagrams of an exemplary modulation components in accordance with one embodiment.
Figure 8 is a block diagram of another exemplary modulation component in accordance with one embodiment.
Figure 9 is a three-dimensional (3D) block diagram of an exemplary modulation component in accordance with one embodiment.
Figure 10 is a three-dimensional (3D) block diagram of an exemplary modulation component in accordance with one embodiment.
Figure 11 is a block diagram of an exemplary method in accordance with one embodiment.
Figure 12 is a block diagram of an exemplary method in accordance with one embodiment.
Figure 13 is a block diagram of an exemplary modulation component variations in accordance with one embodiment.
Figure 14 is a block diagram of exemplary homogeneous range shifting in accordance with one embodiment.
Figure 15 is a graph diagram of exemplary depth dose profiles in accordance with one embodiment.
Figure 16 is a block diagram of an exemplary system in accordance with one embodiment.
Figure 17 is a block diagram of an exemplary implementation of a pseudo code algorithm in accordance with one embodiment.
Figure 18 is a block diagram of exemplary modulation component in accordance with one embodiment.
Figure 19 is a block diagram of an exemplary system in accordance with one embodiment.

### DETAILED DESCRIPTION

Reference will now be made in detail to the various embodiments of the present disclosure, examples of which are illustrated in the accompanying drawings. While described in conjunction with these embodiments, it will be understood that they are not intended to limit the disclosure to these embodiments. On the contrary, the disclosure is intended to cover alternatives, modifications and equivalents, which may be included within the scope of the disclosure as defined by the appended claims. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure.

Presented systems and methods enable efficient and effective radiation planning and treatment, including accurate and convenient transmission of the radiation towards a tissue target. In one embodiment, a workflow for applying an irradiation scheme involving multiple steps is utilized to provide modulation of a particle beam. The workflow includes development of a treatment plan, generating configuration information for a modulation component based on the treatment plan, fabricating the modulation component, and using the modulation component in a system that delivers a radiation dose in accordance with the treatment plan. The modulation component is added to a clinical treatment system (e.g., as an additional component, module, unit, etc.) and features of the modulation component are leveraged by a clinical planning system to modify clinical treatment plans (e.g., for a single-beam-energy application, etc.). Generating configuration information for the modulation component can include various types of information (e.g., geometrical description, material, etc.) related to the modulation component. The workflow can include fabrication/construction of a modulation component according to multiple approaches or schemes, quality control, and the fast application of a particle beam with one accelerator energy.

In one embodiment, a modulation component includes modulation pin cells that provide modulation of a particle beam. Modulation features/characteristics of a modulation pin cell include a) a depth shifting part/portion and b) a distal widening part/portion. An individual modulation component pin cell corresponds to a scan spot/cell position in a scan pattern. Further, a depth shifting part/portion and a distal widening part/portion of the modulation component pin cell are assigned to the scan spot/cell position. For ease of convention, a scan spot/cell is typically referred to as a scan spot. Additional explanations of various features associated with presented modulation systems and methods are set forth in following portions of this description. A particle beam irradiation system is utilized for delivery and application of radiation to a patient. The particle beam irradiation system is capable of performing particle beam delivery in a scan pattern. The particle beam irradiation system is modified (e.g., add a modulation component, etc.) to irradiate a prescribed treatment plan faster than conventional systems (e.g., by increasing a grid spot-to-spot transition time, by increasing a particle beam current, etc.). In one exemplary implementation, a particle beam irradiation system is modified to be able to regulate the beam intensity of each individual scan spot in the scanned field.

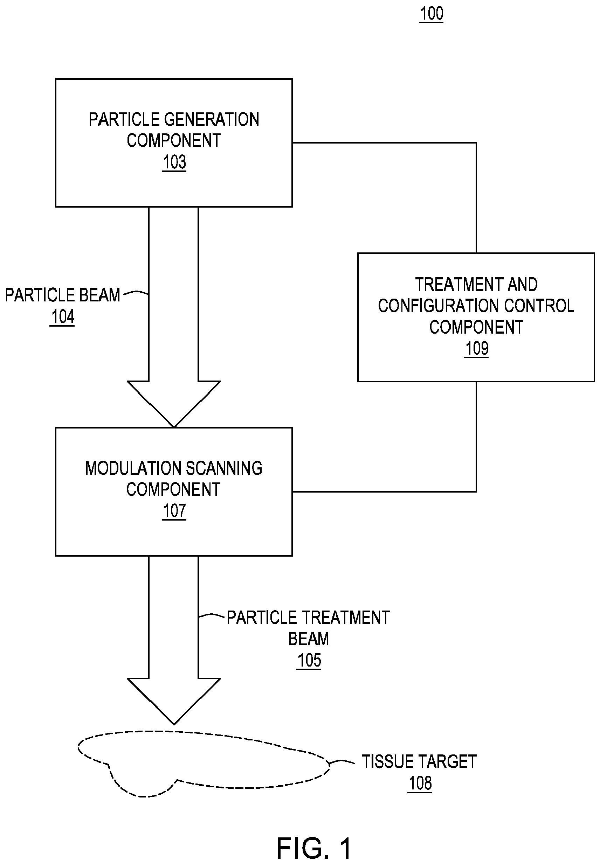

Figure 1 is a block diagram of an exemplary system 100 in accordance with one embodiment. System 100 includes particle generation component 103, modulation scanning component 107, and treatment and configuration control component 109. Particle generation component 103 generates a particle beam 104. Modulation scanning component 107 controls movement of the particle beam in a scan pattern and modulation of the particle beam, resulting in a modulated particle treatment beam 105. Treatment and configuration control component 109 directs configuration of the modulation scanning component 107 and directs delivery of the treatment particle beam. The configuration of the modulation scanning component and delivery of the particle treatment beam is based upon a treatment plan. The particle beam is generated at a same energy level for a first portion of the scan pattern and a second portion of the scan pattern, and the modulated particle treatment beam is different for the first portion of the scan pattern and the second portion of the scan pattern. In one embodiment, the particle generation component 103 generates a particle beam at a same energy level for the first portion of the scan pattern and a second portion of the scan pattern. In one exemplary implementation, the modulation scanning component 107 modulation/adjustment of the modulated particle treatment beam is different for the first portion of the scan pattern than the second portion of the scan pattern. A range of the particle beam is modulated and the range of the resulting modulated particle treatment beam is different for the first portion of the scan pattern than the second portion of the scan pattern. The particle treatment beam 105 is directed towards tissue target 108 (e.g., tumor, etc.).

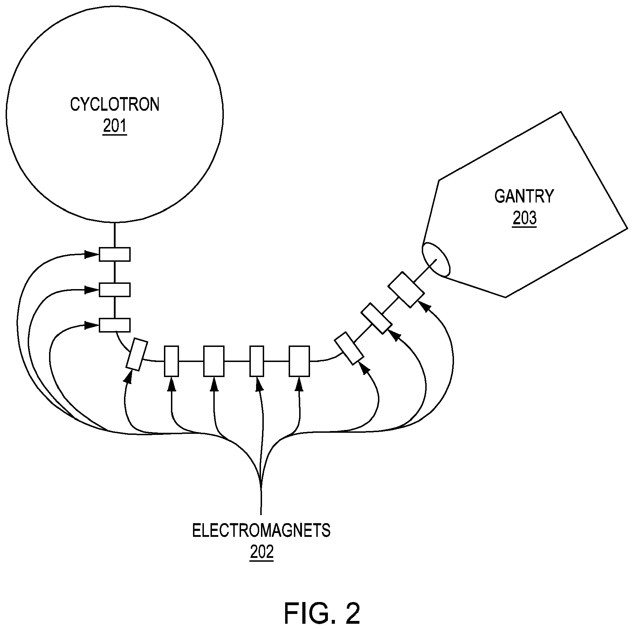

Figure 2 is a block diagram of an exemplary radiation therapy system (e.g., a proton therapy system) in accordance with one embodiment. It is appreciated the present modulation control approaches are applicable to various types of particle therapy systems using protons or ions. For ease of explanation, most of the description herein is directed to proton beams.

Using electric fields, a cyclotron 201 accelerates protons which are then focused, shaped and directed by a beam transport system comprised of electromagnets 202 as a proton beam towards the gantry 203. This proton beam is then guided to the patient using the rotating gantry 203.

In one embodiment, the cyclotron 201 generates a proton beam at a same energy level for a first portion (e.g., first scan spot, first scan gird position, a first set of scan spots, etc.) of the scan pattern and a second portion (e.g., second scan spot, second scan gird position, a first set of scan spots, etc.) of the scan pattern. In one exemplary implementation, the modulation scanning component adjustment of the treatment particle beam (e.g., treatment proton beam) is different for the first portion of the scan pattern than the second portion of the scan pattern. The modulation scanning component adjustment of the treatment proton beam applies fields with many Iso-Energy-Slices (IES) and the treatment beam is at the same energy level for a first portion of the scan pattern and a second portion of the scan pattern. In one exemplary implementation, the treatment fields are irradiated as single IES fields by the treatment beam.

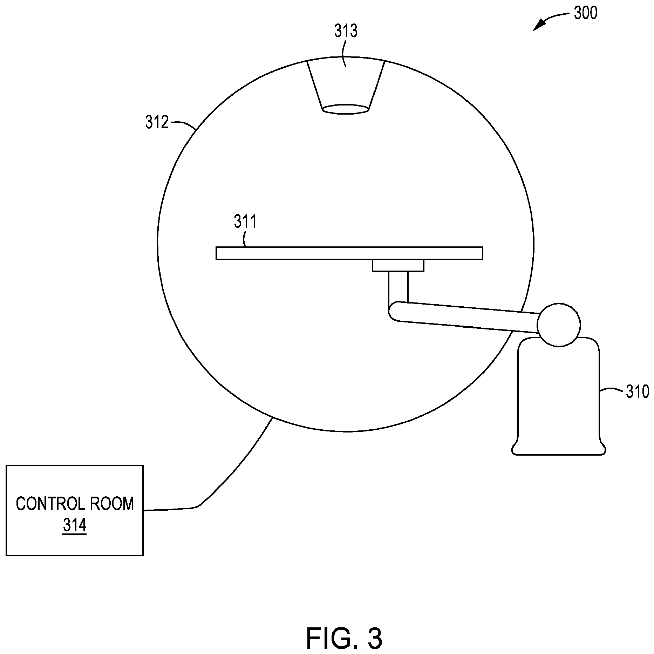

Figure 3 illustrates a block diagram of an exemplary radiation treatment system 300 in accordance with one embodiment. Stand 310 supports a treatment couch 311 upon which the patient lies. A rotatable gantry 312 has a treatment head 313. The treatment head 313 may extend into the gantry 312. The treatment head 313 emits the proton treatment beam. A control system (e.g., a computer system) in the control room 314 controls the entire treatment plan.

An exemplary proton therapy system is the Varian ProBeam^{®} radiotherapy system, commercially available from Varian Medical Systems, Palo Alto, CA.

In one embodiment, a treatment plan indicates a proton particle beam is to be applied in accordance with a scan pattern (e.g., fields with many IES are typically desirable in raster-scan approaches, etc.). A treatment plan calls for proton particle beam transmission to be split into one or more "fields". Each field is in general applied from a specific angular direction into the tissue target region inside the patient. Each field is "sliced" during the planning process into typically equidistant layers of equal energy. In one exemplary implementation, fields typically include approximately 10 to 90 slices. In ion (e.g., heavy ion) plans, the number of slices is typically higher. Attempting to implement a treatment plan requiring many fields with multiple slices per field is traditionally problematic. However, utilizing presented modulation component systems and methods enables fields with many IES to be applied with one energy, unlike traditional approaches. Thus, presented modulation component systems and methods treatment fields are considered effectively irradiated as single-IES fields, drastically reducing the application time of each field compared to conventional systems and methods. In one exemplary implementation, applying one accelerator energy to each field helps avoid time dominating/consuming delays/pauses associated with multiple energy changes. Instead of using many IES, one energy from the beam-generating device is used and the novel system/irradiation process can thus be described as combining 10 to 90 IES into one IES. By this combination a dramatic reduction in treatment time is achieved. In one embodiment, one distinct particle energy is applied to each slice and the slices are called Iso-Energy-Slices (IES).

It is appreciated the presented modulation component approach is flexibly utilized with different treatment plan scenarios. In one embodiment, the approach of applying a single energy particle beam for one plurality of scan spot positions is compatible with applying a different energy to another different plurality of scan spot positions. In one exemplary implementation, even though a single energy is applied to a slice, for another slice the energy is changed. In one embodiment, a first energy is applied to a first set of scan spots and a second energy is applied to a second set of scan spots. In one exemplary implementation, the delivery/application of the particle treatment beam is stopped/paused during energy changes. In one exemplary implementation, a particle beam at a first energy level is applied to a first 25% of the scan spots in a scan pattern, a particle beam at a second energy level is applied to a second 25% of the scan spots in a scan pattern, and a particle beam at a third energy level is applied to the remaining 50% of the scan spots in a scan pattern. Thus, even if it is desirable in a particular situation to have changes between the three energy levels (and potential two pauses), there can still be a significant improvement over many more repeated changes and delays typical in traditional systems and methods.

In one embodiment, a modulation component is utilized in a particle therapy system and method using a scan approach. In one exemplary implementation, a treatment plan includes transmitting the particle beam in a scan pattern. The concept of laterally distributed scan spots is utilized to form the scan pattern. On each slice, many scan spot positions (e.g., called spot-positions, etc.) are typically located on a regular grid (e.g., quadratic grid, as set by the planning system, etc.). In one embodiment, a thin pencil-like beam is deflected by electro-magnets in both directions transversal to the beam to reach planned beam positions in a slice. At each scan spot position on each slice, the beam is kept at constant position. Due to the static beam, a radiation dose is built up over time. After the prescribed dose for the scan spot is applied, the beam is steered to the next scan spot position by the deflecting magnets. Deflector magnets and their power supplies are optimized to minimize the transition time between scan spot positions. In one exemplary implementation, relatively mild improvements of the irradiation time per scan spot position and the spot-to-spot timing of a factor 2 to 10 are reached, and the required increase in speed of treatment of a factor 100 to 1000 are reached. In one embodiment, FLASH requirements of increasing the dose rate by a factor of 10 to 1000 are met by a modulation component system and method, unlike conventional approaches. In addition to scanning a particle beam laterally to many scan spot positions, homogenous and field individual presented modulation components are set to allow a conformal irradiation with one single energy of the irradiation system.

Unlike traditional approaches, FLASH-therapy regime can effectively be utilized with a scan radiation scheme. In one embodiment, since there are no/fewer energy changes in a modulation component approach when a particle beam is transitioning through scan spot locations/positions, there is no/fewer corresponding pauses or stops and a system can deliver relatively high dose rates including FLASH dose rates (e.g., FLASH dose rates of 20-40 Gy delivered in less than one second, and as much as 120 or more Gy per second). In one exemplary implementation, scan spot positions are arranged in a grid within a scan pattern. A grid approach is capable of or can present an exemplary scan pattern setup in a schematical way.

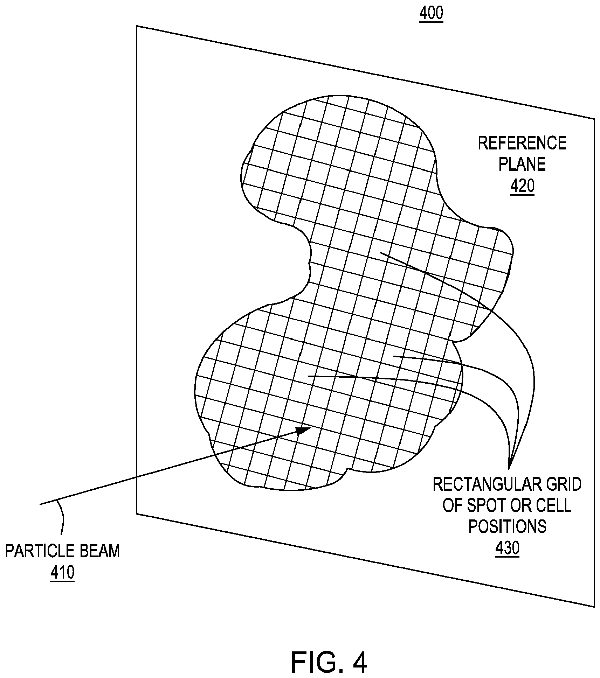

Figure 4 is a block diagram of an exemplary scan pattern 400 in accordance with one embodiment. Scan pattern 400 can correspond to a target grid. In one exemplary implementation, scan pattern 400 is associated with a raster scan. The scan pattern 400 is implemented in a single layer irradiation approach. The scan pattern 400 can include a grid of scan spot/cell positions 430 that correspond to target grid positions in a target tissue field slice. Particle beam 410 is directed/guided/steered in a scan sequence (e.g., raster scan, line scan, spiral scan, etc.) such that a particle beam moves or traverses the scan spot/grid cell positions 430. The scan spot positions 430 are associated with discrete positions on the reference plane 420 that is inside or close to the patient. In one embodiment, the reference plane corresponds to a field slice. In one exemplary implementation, the scan spot positions and grid follow the contour of a target tissue treatment area.

In one embodiment, a first portion of the scan pattern can correspond to a scan spot, a plurality of scan spots, a part of a scan spot, and so on. In one embodiment, a second portion of the scan pattern can correspond to a different scan spot, a different plurality of scan spots, a different part of a scan spot, and so on.

The shape of scan spots/cells can vary (e.g., regular quadratic, rectangular, triangular, hexagonal shape, etc.). In addition, the scientific method of "Penrose tiling", using quasi regular 2D grids including very few 2D basic shapes, is applied.

A modulation component can adjust or modulate a particle beam. In one embodiment, for a lateral scan spot position in the field, the range modulator shifts the largest deposition depth to lower depths and generates a homogenous dose (or a desirable dose profile determined by an optimization algorithm) from largest penetration depth to smallest depth. Also, some considerations are made for maximizing the local dose rates within the field through an understanding of the delivery mechanics. In one embodiment, a modulation component includes a plurality of modulation pin cells. In one exemplary implementation, a modulation component pin cell comprises a) a depth shifting part/portion and b) a distal widening part/portion. An individual modulation component pin cell can correspond to a scan spot position, and a depth shifting part/portion and a distal widening part/portion are assigned to the scan spot position.

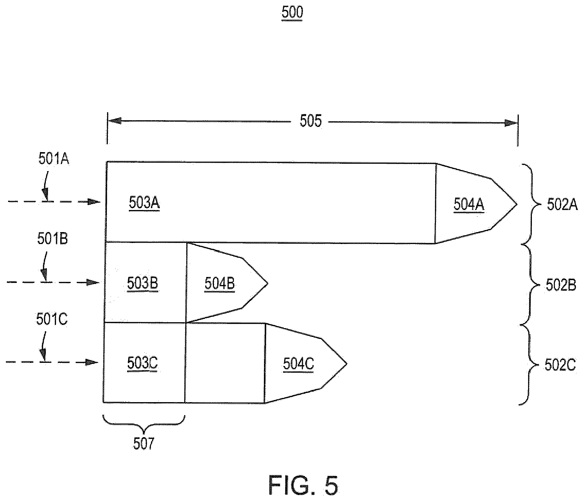

Figure 5 is a block diagram of exemplary modulation component 500 in accordance with one embodiment. The modulation scanning component 500 is partitioned into a plurality of modulation component pin cells in which a first modulation pin cell of the plurality of modulation pin cells and a second modulation pin cell of the plurality of modulation pin cells have different configurations. The modulation component 500 includes modulation pin cell 502A, modulation pin cell 502B, and modulation pin cell 502C. In one embodiment, a modulation pin cell corresponds to a scan spot or scan cell position is a scan pattern. Modulation pin cell 502A includes modulation pin cell first portion 503A and modulation pin cell second portion 504A. Modulation pin cell 502B includes modulation pin cell first portion 503B and modulation pin cell second portion 504B. Modulation pin cell 502C includes modulation pin cell first portion 503C and modulation pin cell second portion 504C. Particle beam 501 is directed towards the modulation pin cells. For ease of explanation, particle beam 501 is shown as 501A, 501B, and 501C to illustrate the particle beam being directed to the different modulation pin cells 502A, 502B, and 502C, respectively.

In one embodiment, a first modulation pin cell of the plurality of modulation pin cells 502A includes a first portion 503A with a first density and a second portion 504A with a second density, and a second modulation pin cell of the plurality of modulation pin cells 503B includes a first portion 503B with a first density and a second portion 504B with a second density. In one exemplary implementation, the first density is the same value respectively in the first modulation pin cell of the plurality of cells and the second modulation pin cell of the plurality of cells. A dimension (e.g., first length, first width, cross-section area, etc.) of the first portion in the first modulation pin cell of the plurality of modulation pin cells is different than a respective dimension (e.g., second length, second width, second cross-section area, etc.) in the first portion of the second modulation pin cell of the plurality of modulation pin cells. In one exemplary implementation, a first shape (rectangle) of the first portion in the first modulation pin cell of the plurality of modulation pin cells is different than a second shape (pyramid) in the second portion of the first modulation pin cell of the plurality of modulation pin cells.

The modulation pin cells can have various configurations. In one embodiment, modulation pin cells have a pin cell volume configuration. The particle beam 501 (e.g., A, B, and C) impinges on the modulation pin cells 502A, 502B, and 502C. In one embodiment, the modulation pin cells are arranged in a grid. In one exemplary implementation, along the beam axis, modulation pin cells (e.g., 502A, 502B, 502C, etc.) include a first portion/region (e.g., 503A, 503B, 503C, etc.) which is solid (totally filled) and a second portion/region which is partially filled (e.g., 504A, 504B, 504C, etc.). The totally filled region of a modulation pin cell (e.g., 503A, 503B, 503C, etc.) lowers the beam energy, thus decreasing the total range of particles. The partially filled region (e.g., 504A, 504B, 504C, etc.) shape the depth dose profile of the particle beam over a depth, given by the height of the partially filled part. The total extension (e.g., 505, etc.) of the modulation component cannot be larger than the maximal energy of the beam. A modulation component may consist of a lower, plate-like portion or region (e.g., 507, etc.), where all cells are filled.

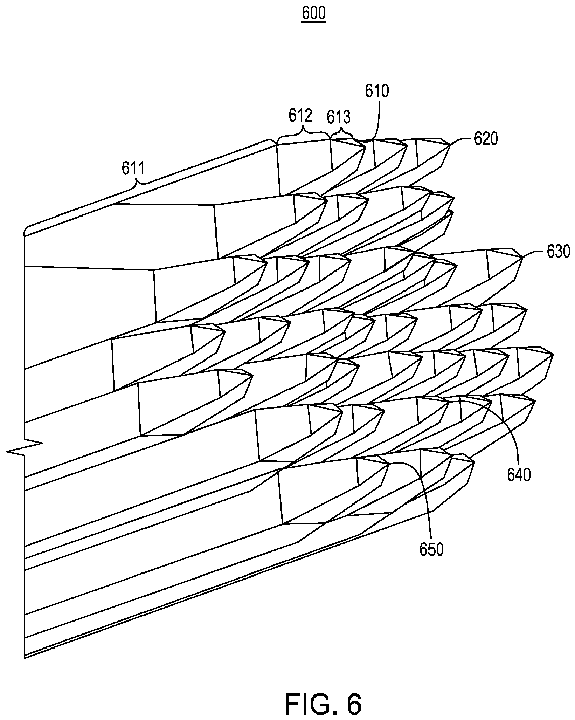

Figure 6 is a three-dimensional (3D) block diagram of exemplary modulation component 600 in accordance with one embodiment. Modulation component 600 includes modulation pin cells (e.g., 610, 620, 630, 640, 650, etc.). In an exemplary implementation, a first modulation pin cell of the plurality of cells 610 includes a first portion 611, a second portion 612, and a third portion 613. The first portion 611, a second portion 612, and a third portion 613 can have different configurations (e.g., length, shape, etc.).

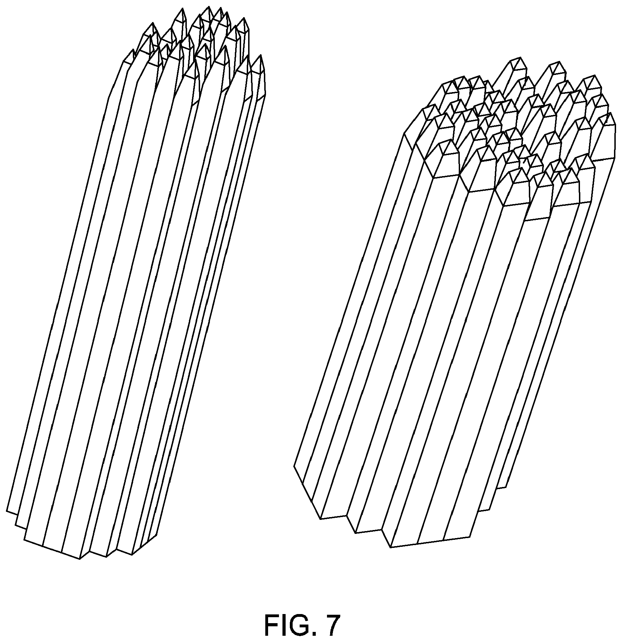

Figure 7 includes exemplary three-dimensional (3D) block diagrams of exemplary modulation components in accordance with one embodiment. The 3D block diagrams show the modulation components from different perspectives.

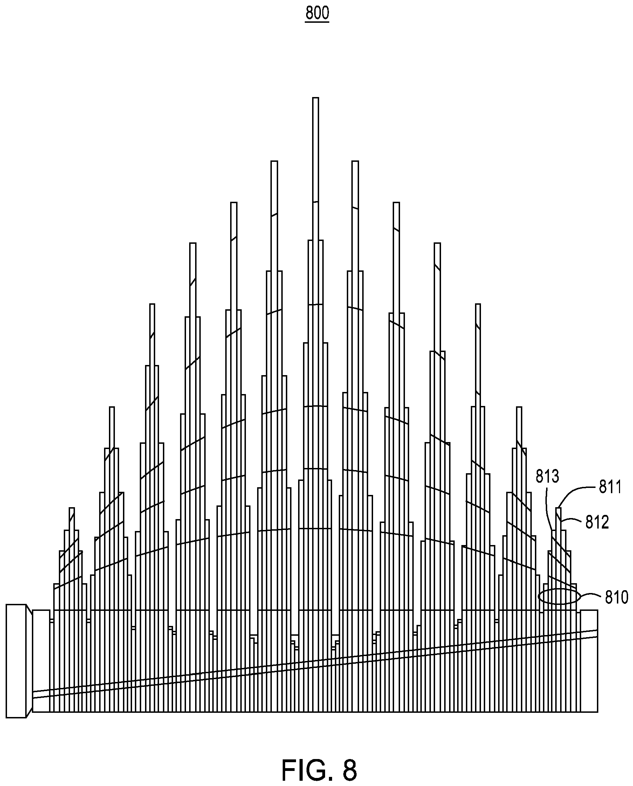

Figure 8 is a block diagram of another exemplary modulation component 800 in accordance with one embodiment. In one embodiment, a plurality of modulator component cells can correspond to a scan spot position. In one exemplary implementation, a set/group of modulator component pin cells correspond to a scan spot. The modulator component cells (e.g., 811, 812, etc.) within the set or group can have the similar configurations (e.g., 812, 813, etc.) or different configurations (e.g., 812, 811, etc.).

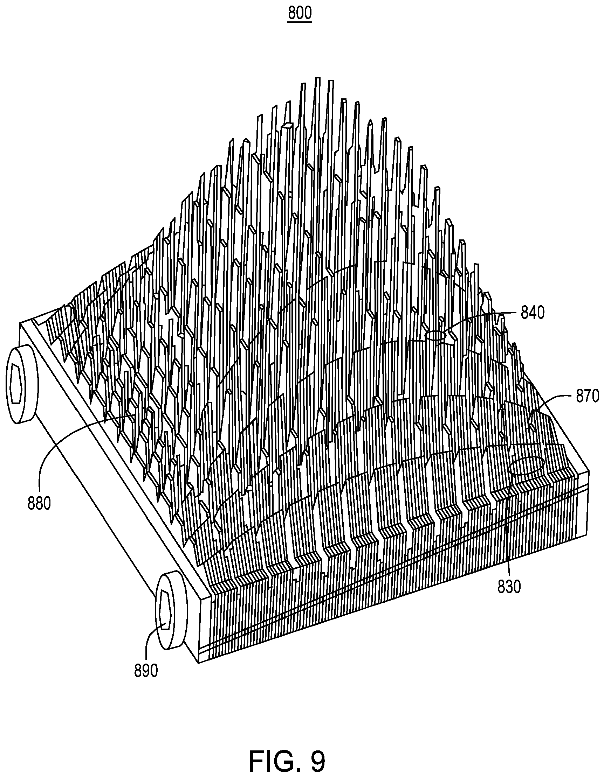

Figure 9 is a three-dimensional (3D) block diagram of exemplary modulation component 800 in accordance with one embodiment. The sets/groups of modulator component cells can have similar configurations or different configurations. In one exemplary implementation, modulator component cells set/group 830 has a wider configuration collectively than modulator component cells set/group 840. In one exemplary implementation, modulator component cells set/group 830 (e.g., 9 modulation pin cells, etc.) has a different number of modulation pin cells than modulator component cells set/group 830 (e.g., 6 modulation pin cells, etc.). In one exemplary implementation, modulation pin cell 870 (e.g., relatively pointy pyramid type top, etc.) has a different configuration than modulation pin cell 880 (e.g., relatively flat rectangular type top, etc.).

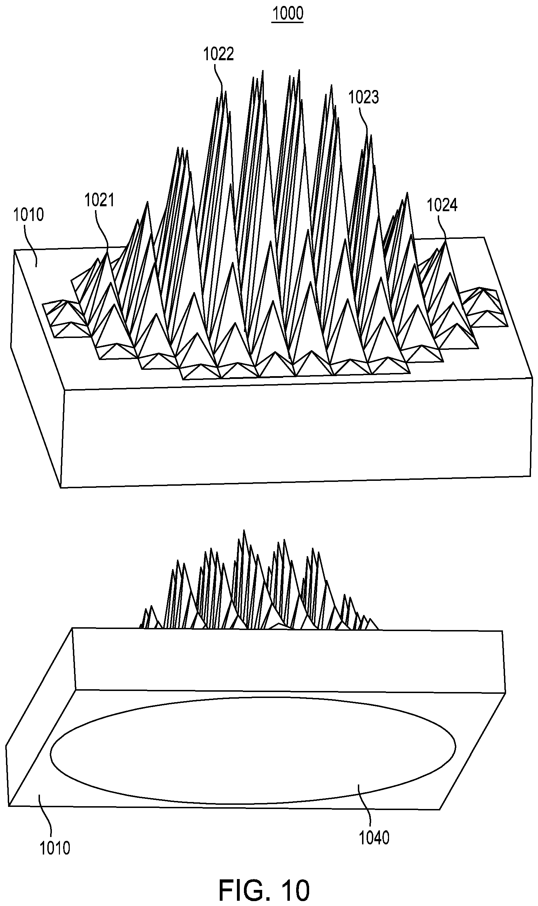

Figure 10 is a three-dimensional (3D) block diagram of modulation component 1000 in accordance with one embodiment. Modulation component 1000 includes a base portion 1010. Base portion 1010 is coupled to modulation pin cell 1021, modulation pin cell 1022, and modulation pin cell 1023, and modulation pin cell 1024. The modulation component 1000 can have a scan pattern area 1040 through which particle beams propagate towards the modulation pin cells.

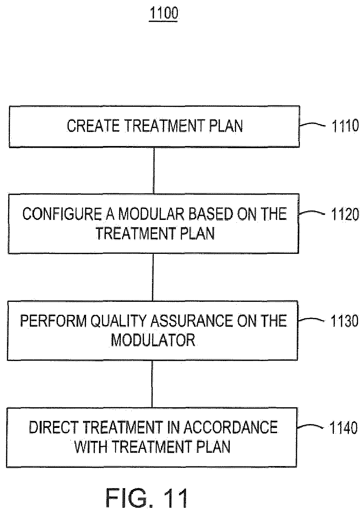

Figure 11 is a block diagram of an exemplary method 1100 in accordance with one embodiment.

In block 1110, a treatment plan creation process is performed in which a treatment plan is created. Portions of the treatment plan creation/development are automated. In one exemplary implementation, a treatment plan is created utilizing various algorithms (e.g., in a computer system, etc.).

In block 1120, a modulation component configuration process is performed. In one embodiment, a modulation component is configured based on the treatment plan.

In block 1130, a quality assurance process is performed on the modulation component.

In block 1140, a treatment is performed. In one embodiment, the treatment is performed in accordance with the treatment plan.

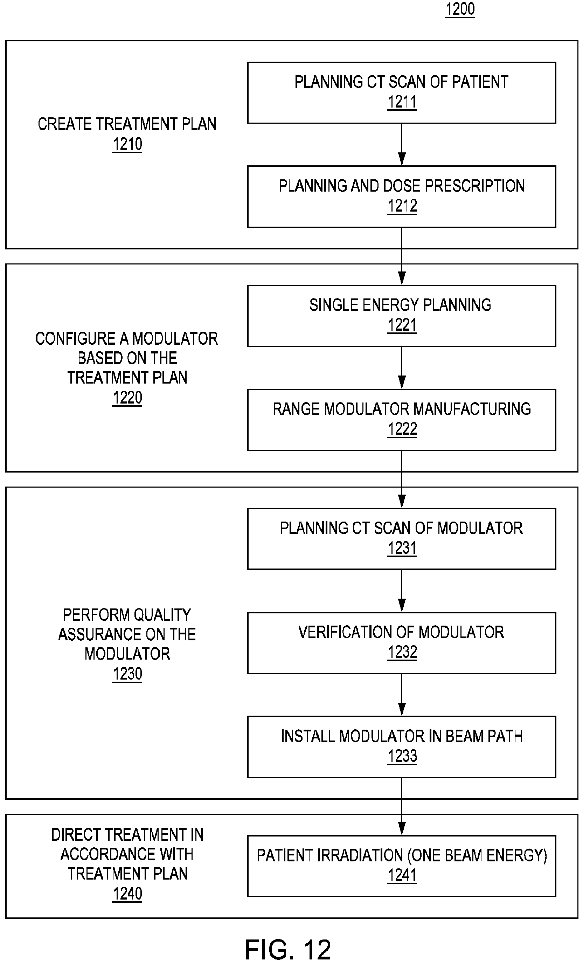

Figure 12 is a block diagram of an exemplary method 1200 in accordance with one embodiment.

In block 1210, a treatment plan creation process is performed in which a treatment plan is created. In one embodiment, the treatment plan creation process includes acquiring a planning CT scan (a CT scan used for treatment planning) of a patient (e.g., block 1211, etc.). A treatment plan creation process can include a dose prescription (e.g., block 1212, etc.) and treatment planning based at least in part upon the results of the CT scan of the patient. In one embodiment, a radiological 3D image of the treatment region within a patient is taken, which uses an X-Ray computer tomograph. In one exemplary implementation, the planning CT is similar to conventional "planning CT" approaches. Planning of the target region and dose description, as well as determination of appropriate one or more fields, can include using an industrial oncology planning system. The planning system is modified to plan for laterally distributed scan spot positions (e.g., similar to scan pattern 400 illustration in Figure 4, etc.) using a particle beam at one energy level (e.g., typically the highest available energy of the system). The result of this process step is a plan (e.g., spot list, etc.) for a modulating component. The modulating component is referred to as the "range modulator".

In block 1220, a modulation component configuration process is performed. In one embodiment, a modulation component is configured based on the treatment plan. In one exemplary implementation, a modulation component configuration process includes developing a modulation component configuration (e.g., block 1221) compatible with single energy generation of particle beams that are directed at the modulation component. A modulation component configuration process can include fabricating/constructing/manufacturing of a modulation component (block 1222). For ease of explanation, the term fabricated is used to include constructing, building, manufacturing and so on.

It is appreciated a modulation component can be fabricated in various ways. In one embodiment, the modulation component is fabricated as a physical object of any appropriate rigid material. In one exemplary implementation, there is a known relationship of unit depth in the modulation pin cell to the radiation characteristics of a corresponding unit length in a particular material. In one embodiment, the ratio of one unit-depth of the material compared to the same unit-depth of water is known. Since the planning systems typically compute penetration depths of beams in water-equivalent depth, this ratio of the material gives a linear scaling factor to the height of the range modulator.

In one embodiment, a fabrication process has a high enough resolution to realize the prescribed geometry well enough and avoids unwanted material inclusion (including voids) in the solid parts. In general, the modulation component is made by several technical processes, which are automatized and computer controlled, like computer-controlled drilling and milling, cutting or erosion. In one exemplary implementation, a preferred realization uses additive manufacturing techniques based on the polymerization of liquid plastics. In one embodiment, a specific manufacturing technique can be chosen freely.

The CT scan of the field-individual parts of a modulation component (1231) and the results of the initial planning process (e.g., block 1210, etc.) are used in an extended (or separate quality assurance module) of the planning software. This module allows for import of the CT scan of the modulation component plan, to reorient the CT scan and overlay it to the planned beam path of the corresponding patient-field. Since the planning system is specialized to determine the beam path of the treatment beam passing through different materials/tissues, the "action" of the 3D scanned modulation component species is studied within the planning system. If deviations to the prescribed dose distribution are found, the modulation component is exchanged by a more appropriate one. In this way, relevant manufacturing deviations are minimized or excluded. Moreover, with knowledge of the delivery properties of a spot list, such a quality assurance module may verify dose rate distributions predicted by the treatment planning system (TPS).

In block 1230, a quality assurance process is performed on the modulation component. A quality proof of the local field-specific manufactured parts is included. In one embodiment, for the treatment of the patient, simply the parts of the modulation component, corresponding to each treatment field, are brought into the beam path via an appropriate mounting system. Specific emphasis is spent on the proof that a given range modulator was correctly manufactured. In one embodiment, quality assurance process includes performing a simulated CT scan of the modulation component created in block 1220. Clinical planning-CT scanners are utilized. In one exemplary implementation, quality assurance of a modulation component is checked by scanning it with a clinical planning X-Ray CT approach modified with presented modulation component novel quality assurance approaches to verify (e.g., block 1232, etc.) the modulation component. A verified modulation component is left installed (e.g., block 1233) in a particle treatment beam path for radiation treatment of the patient. If a modulation component is not verified as reliable and meeting treatment plan requirements various corrective actions are taken (e.g., the modulation component is removed, the treatment plan altered, etc.).

It is appreciated that various aspects of CT scanning approaches are implemented in a quality assurance process. In one embodiment, a dual energy CT scanner is used for the planning CT to improve accuracy of the overall process. The quality control is directed to individual elements. In one exemplary implementation, each field-specific manufactured element is quality controlled by scanning it with a 3D scanning technique, resolving its internal composition in a non-destructive way, preferably using X-Ray scanners. The individually manufactured elements may also be scanned with MRI or ultrasound techniques. The individually manufactured elements are scanned with a clinical planning CT scanner (e.g., which may be available at the user's clinical installation, etc.).

In one embodiment, various aspects of the presented modulation component quality assurance approaches (e.g., usage of X-Ray CT scanners, using multiple X-Ray energies, allowing to work with automated material decomposition of scanned objects, etc.) can have numerous beneficial impacts. In a variant of the quality-check, "photon counting CT scanners" are used, which typically have a much higher resolution than conventional clinical CT-scanners and also allow an improved automated material decomposition of scanned objects. In one exemplary implementation, resulting 3D scans are imported to a software module that "allows" for virtual passage of a particle beam through individual elements of the modulation component to ensure that the anticipated dose distribution is achieved.

The quality control can use a clinical CT-scan and a comparison to qualify each modulation component. The comparison is automatically performed (e.g., by a quality control process, etc.). Results from the CT scan of the field-individual parts are checked by a clinical planning system or by an external application. Thus, in one embodiment, the CT scan-data is used in a digital-twin-like method to ensure the correct dose distribution by using the individual modulation component.

In block 1240, a treatment is performed. In one embodiment, the treatment is performed in accordance with the treatment plan created in block 1210 utilizing the modulation component fabricated in block 1220 and quality checked in block 1230. In one exemplary implementation, a particle beam is generated at a single energy level for multiple scan spot positions. The particle beam is modulated on a scan spot position basis by modulation component. A particle (e.g., proton or ion) beam is applied with its maximal (typically highest intensity) energy and laterally scanned to the prescribed lateral scan spot positions. In one exemplary implementation, the depth modulating cells of the modulation component ensure that the prescribed 3D-depth dose distribution is applied according to the prescription.

In one embodiment, adjustment/modulation of the treatment beam and radiation by the modulation scanning component optimizes avoidance of detrimental impacts to non-target tissue. In one exemplary implementation, the adjustment of the treatment beam and radiation by the modulation scanning component spares non-target tissue from detrimental impacts in a selectively granular manner.

It is appreciated there are various approaches to configuring a modulation component. The modulation component is made by milling, drilling or erosion techniques. In one embodiment, the modulation component is additively manufactured (e.g., 3D printing, etc.). The modulation component is made from a PMMA-like plastic material but might also be made of any other appropriate material, like other plastics or metals or mixtures thereof. The modulation component is made by a polymerization process from liquid polymers.

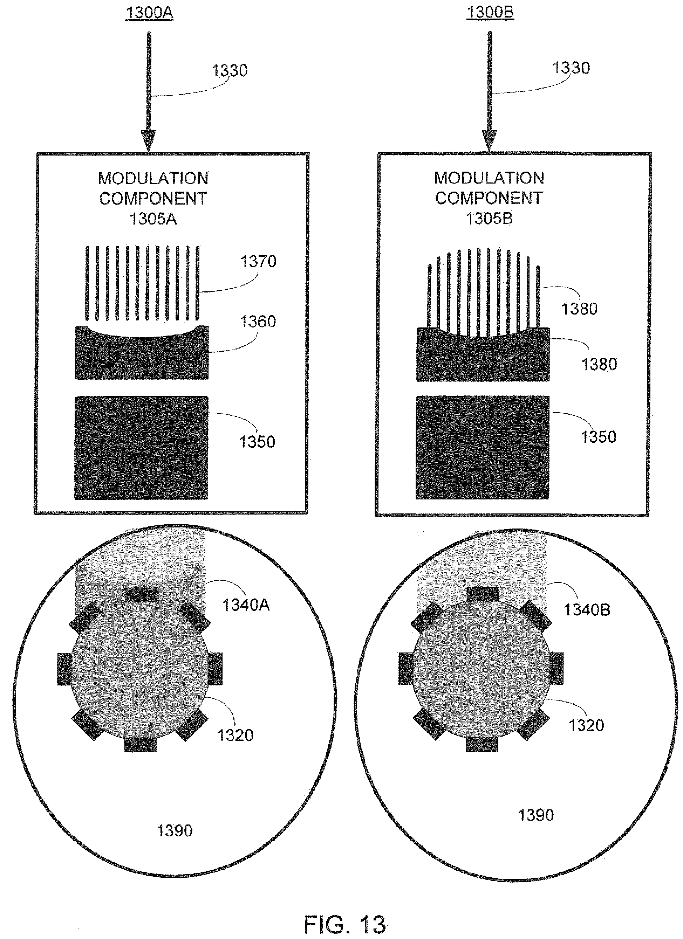

It is appreciated a modulation component can have various configurations. Figure 13 is a block diagram of exemplary modulation component variations in accordance with on embodiment. In both variants 1300A and variant 1300B a patient 1390 is irradiated at a prescribed volume 1320 by a radiation field shaped to a prescribed 3D dose distribution as well as possible. To achieve this, a particle beam 1330 is sent through the respective range modulation components 1305A and 1305B. One part of the range modulation components includes a homogenous plate 1350 to adapt the penetration depth to a maximal value. In variant 1305A a field-individual shaping element 1360, which ensures that the prescribed penetration depth is matched at each lateral position, together with a depth dose widening element 1370, is utilized to spread out the depth dose to full target extension. In variant 1305B one field-individual range modulation component 1380 is made for matching prescribed penetration depth and prescribed flat depth dose for each lateral position. Variant 1305A has a radiation field 1340A and variant 1305B has a radiation field 1340B. The shape of the radiation field originates from the differently shaped components (1370 / upper half of 1380). For both 1305A and 1305B, the shaping element and the range modulation component may be one single component or split into separate parts without influencing the dose distributions.

In one embodiment, the selection of a modulation component configuration approach or scheme and a particular variant is important. With respect to variants 1305A and 1305B, in both, the range modulator is located between the beam outlet and the patient. In addition, they may include a planar part, shifting the maximal penetration depth of the beam near to the distal end of the prescribed dose distribution. In variant 1305A the depth and range modulation is achieved by a lateral homogenous ridge filter 1370, widening the depth dose distribution of the particles of scan spot positions to the maximal needed amount. A second part of the modulation component is field-individual and shifts the penetration depth of the beam to the prescribed maximal depth. Due to the common widening of spots to the largest extent of the tumor in beam direction, such a uniform modulation component (ridge-filter) is reused for other patients. In one embodiment, additional consideration is given to determining a limit on the number of reuses as continued multiple re-use can lead to a reduced target dose conformality.

In variant 1305B the spot-individual depth-widening and depth-shifting element are combined into one field-individual modulation component. Due to the spot-individual widening of the beam, the dose conformity to the target is improved. In one embodiment, a modulation component may selectively only adjust the penetration depth at each individual scan spot position, accompanied by a laterally flat beam widening element (e.g., similar to modulation component 1300A, etc.). In one embodiment, a modulation component can include individual range and width modulating elements, determining the depth-dose distribution (e.g., optionally designed by a dose optimization algorithm, etc.) at individual scan spot positions (e.g., similar to modulation component 1300B, etc.).

In one embodiment, a modulation component is considered a range modulator. In addition to range modulation by the modulation component itself, a modulation component is used with an overall range shifting element. The overall range shifting element can include a "plate" or set of "plates". In one embodiment, a range shifting element selectively absorbs a portion of the particle beam energy. The overall range shifting element can comprise multiple wedges, mounted in a way that they are individually adjusted to achieve a variable controlled range-shifting depth. In one embodiment, selection and control of the range-shifting depth is automatically directed by a control component (e.g., 109, etc.). The range-shifting depth is selected and controlled in accordance with a treatment plan.

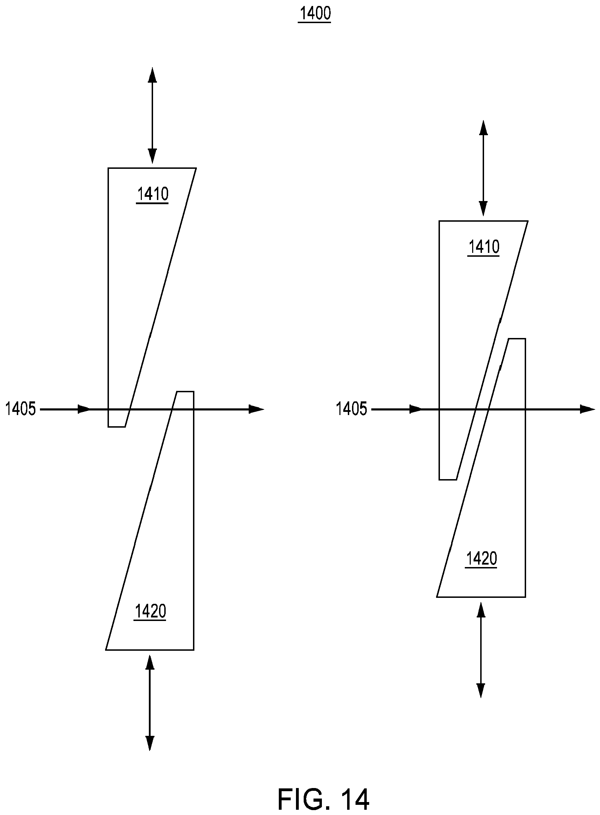

Figure 14 is a block diagram of exemplary homogeneous range shifting in accordance with one embodiment. A double wedge combination of 1410 and 1420 are used and the particle beam 1405 is sent through a first wedge 1410, followed by a second wedge 1420. Both wedges are moved transversely to the beam, with movements synchronized. By this construction, the beam is running through an absorber of variable thickness.

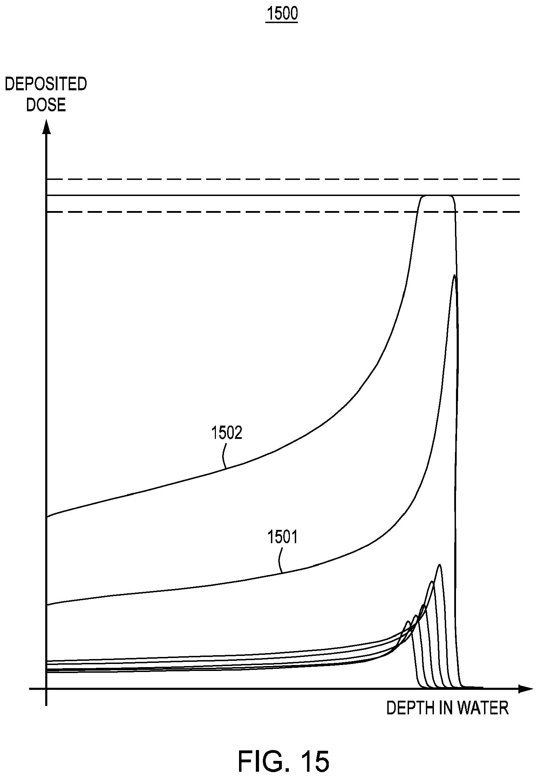

In one embodiment, a very conformal dose distribution is built-up inside a tissue target volume by overlaying pencil particle beams sent to various depths by modulation of a single energy beam and a modulation component adjusting the beam energy. The applied doses can sum up to a conformal (homogenous) dose as depicted in Figure 15. Figure 15 is a graph diagram of exemplary depth dose profiles in accordance with one embodiment. The graph X axis indicates the depth in water and the graph Y axis indicates deposited dose. A beam entering water-equivalent material from the left, deposits dose along its path, until reaching the end of its range. The depth-dose distribution for a single energy particle, called Bragg-peak (e.g., 1501, etc.) is relatively sharp. Particles of a multitude of energies (here six) with individual intensities are overlaid to reach a homogeneous depth dose distribution (e.g., 1502, etc.). In one embodiment, the presented approach or scheme leverages the mechanisms of stopping the particles in the tissue, since heavy particles deposit most energy per penetrated path-length near the end of their travel range in tissue (e.g., a Bragg peak, as depicted Figure 15).

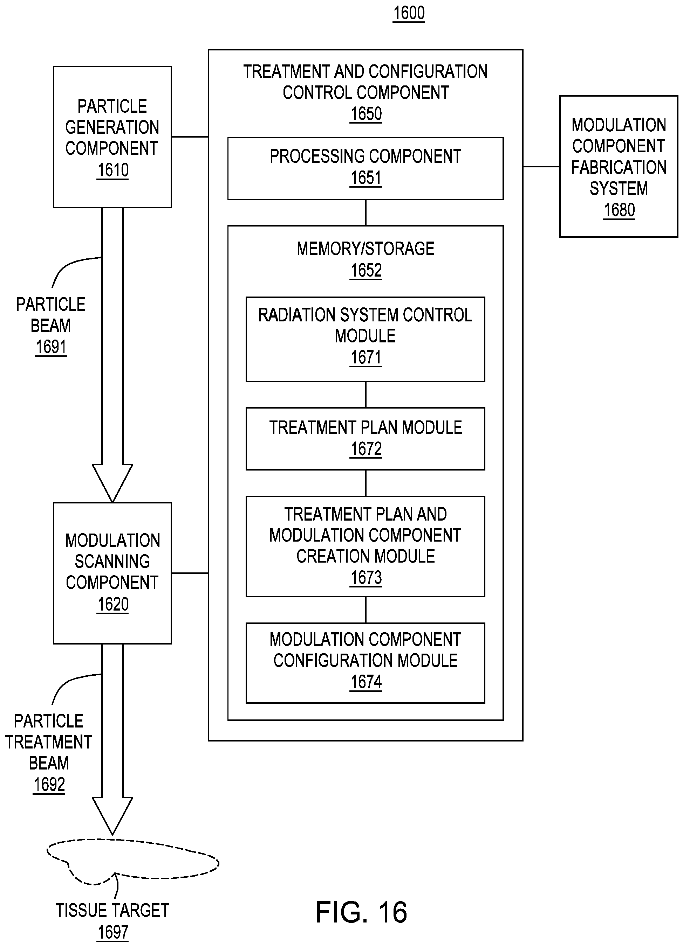

Figure 16 is a block diagram of an exemplary system 1600 in accordance with one embodiment. System 1600 includes particle generation component 1610, modulation scanning component 1620, treatment and configuration control component 1650, and modulation component fabrication system 1680. Particle generation component 1610 generates a particle beam 1691. Modulation scanning component 1620 controls movement of the particle beam in a scan pattern and modulation of the particle beam resulting in a modulated particle treatment beam. Treatment and configuration control component 1650 directs configuration of the modulation scanning component and directs delivery of a treatment particle beam. It includes a processing component 1651, and a memory/storage 1652. A radiation system and control module 1671, treatment plan module 1672, treatment plan and modulation component creation module 1673, and modulation component configuration module 1674 are stored in memory/storage 1652. Configuration of the modulation scanning component and delivery of the treatment beam are based upon a treatment plan, wherein the particle beam is generated at the same energy for a first portion of the scan pattern and a second portion of the scan pattern, and the modulated particle treatment beam is different for a first portion of the scan pattern and a second portion of the scan pattern. The range of the particle treatment beam is different for a first portion of the scan pattern and a second portion of the scan pattern.

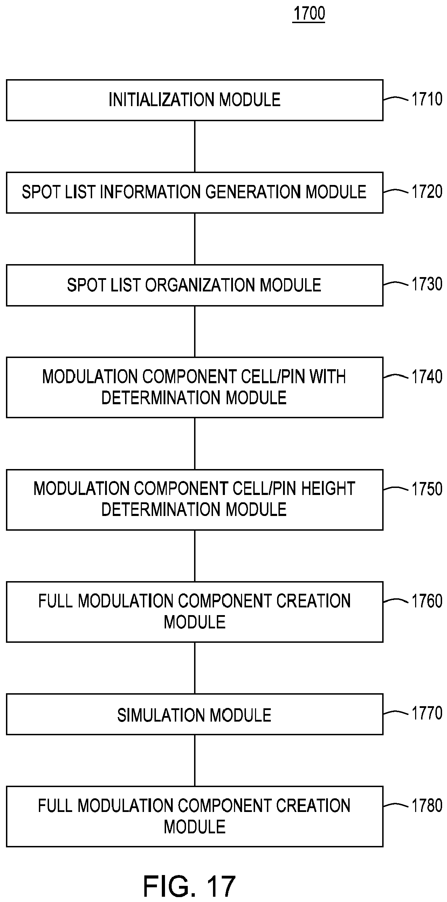

Figure 17 is a block diagram of an exemplary implementation of a pseudo code algorithm in accordance with one embodiment.

The pseudo code algorithm begins with an initialization module 1710. In initialization module 1710 a treatment plan is optimized in treatment planning systems according to clinical constraints. The beam data used for the dose calculation mimics the energy variation through range shifter plates at the nozzle exit instead of using the degrader at the accelerator exit. For each treatment field, a spot list results from the optimization, containing the lateral scan spot positions (x and y), the beam energy (E) as well as the weight (W) for each scan spot. In one embodiment, tumors are treated with one to many fields, with the optimization algorithm handling both single-field and multi-field configurations. In one embodiment, the optimization is made in a second computer program, after the clinical planning system has computed the target dose distribution and IES/spot position maps.

Spot list information generation module 1720 generates spot list information. For multiple unique (x,y) positions, scan spots with the same (x,y) position but differing beam energy, E, are collected and combined into a single beamlet. Each beamlet can have a unique (x,y) position and an absolute weight that is the sum of contributing original scan spots. In addition, it contains a list of relative weights of every energy step within the beamlet. In one embodiment, these relative weights are calculated as the absolute weight of every (x,y,E) scan spot divided by the total weight of the beamlet. Furthermore, the energy steps are translated to the thickness of RM material required to degrade the incident beam energy to the intended energy. The incident beam energy is typically chosen as the highest available energy, but it could be any energy that is greater than or equal to the highest energy of a single spot in the treatment plan.

Spot list organization module 1730 organizes the spot list information from module 1720 into a final spot list. The final spot list to be delivered by the machine includes the list of beamlet (x,y) positions and the absolute weight of each beamlet, whereas the delivered energy is the chosen incident energy for every beamlet. The delivered spot list takes care of the lateral modulation of the dose, whereas the physical range modulator shapes the dose along the beam axis.

In the modulation pin cell width determination module 1740, the height configuration of modulation pin cell heights are determined. In one embodiment, the pin cell width defines a regular quadratic grid. Since the grid points don't necessarily align with the beamlet positions, the relative weights per energy are spatially interpolated to the grid points. These interpolated weights per energy are used to determine the modulation pin cell shape at a given grid point. The weights per energy are translated into area fill fractions per pin height. For the lowest energy/highest pin height, the area fill fraction simply corresponds to the relative weight of said energy at the given modulation pin cell position. For the next higher energy, the fill fraction is the sum of the weights of the previous plus the current energy. This is repeated up to the highest energy (corresponding to the lowest pin height), which will have an area fill fraction of 100%.

In the modulation pin cell/pin height determination module 1750, the height configuration of modulation pin cell/pin heights are determined. In this module 1750, the previously determined area fill fractions as a function of the modulation pin cell/pin height need to be converted to a 2D shape (e.g., square, rectangle, circle, etc.) whose area corresponds to a fraction of the pin base area. These 2D shapes together with the respective material thicknesses then define the shape of the pin at the given position.

In full modulation component creation module 1760, the full modulation component is created by combining the pin cells at the scan pattern or grid positions into one object.

In simulation modulation 1770, the delivery information can now be simulated using a Monte Carlo tool. If the resulting dose distribution differs from the planned dose distribution in the treatment planning system by more than a certain tolerance, the design of the range modulator is adapted to correct for these discrepancies. If needed, this step is repeated. The computer program to check the field-specific part of a range modulator is part of the clinical planning system or can be done on a totally different system, to use alternative beam propagation algorithms. This will allow an independent cross check of the computing techniques.

Modulation component configuration download module 1780 directs the download transmission of the delivery information. The modulator is manufactured using the delivery information and is ready for quality assurance.

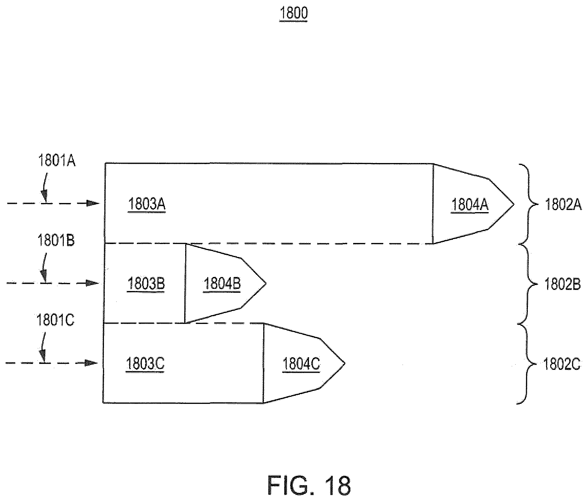

Figure 18 is a block diagram of exemplary modulation component 1800 in accordance with one embodiment. The modulation scanning component 1800 is partitioned into a plurality of modulation component pin cells in which a first modulation pin cell of the plurality of modulation pin cells and a second modulation pin cell of the plurality of modulation pin cells have different configurations. The modulation component 1800 includes modulation pin cell 1802A, modulation pin cell 1802B, and modulation pin cell 1802C. In one embodiment, a modulation pin cell corresponds to a scan spot position in a scan pattern. Modulation pin cell 1802A includes modulation pin cell first portion 1803A and modulation pin cell second portion 1804A. Modulation pin cell 1802B includes modulation pin cell first portion 1803B and modulation pin cell second portion 1804B. Modulation pin cell 1802C includes modulation pin cell first portion 1803C and modulation pin cell second portion 1804C. Particle beam 1801 is directed towards the modulation pin cells. For ease of explanation, particle beam 1801 is shown as 1801A, 1801B, and 1801C to illustrate the particle beam being directed to the different modulation pin cells 1802A, 1802B, and 1802C respectively.

It is appreciated modulation component 1800 is fabricated by various processes. In one embodiment, modulation component 1800 is fabricated locally in the field. In one embodiment, modulation component 1800 is fabricated remotely. In one embodiment, modulation component 1800 is partially fabricated remotely and partially fabricated locally. Field-specific range-modulation elements are combined with universal elements to minimize the number and size of field-individual parts. In one exemplary implementation, the modulation pin cell 1802A, modulation pin cell 1802B, and modulation pin cell 1802C are remotely fabricated individually and coupled together locally in the field. Similarly, parts of a modulation pin cell 1802A, 1802B, and 1802C are remotely fabricated and coupled together locally in the field. Modulation pin cell first portions 1803A, 1803B, and 1803C are fabricated locally and modulation pin cell second portions 1804A, 1804B, and 1084C are fabricated remotely. The clear or white part of modulation pin cell first portions 1803A, 1803B, and 1803C are fabricated remotely and shaded or grey part of modulation pin cell first portions 1803A, 1803B, and 1803C are fabricated locally.

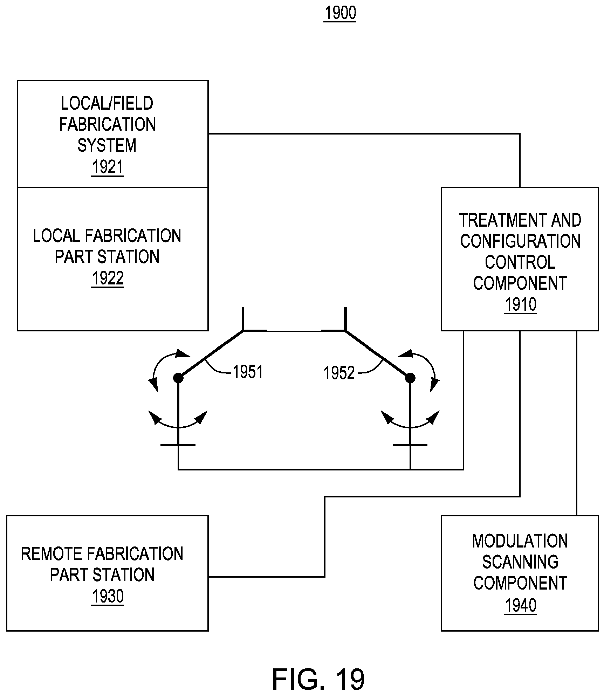

In one embodiment, universal and field-specific parts of the modulation system are moved by automatized systems, to minimize user interaction inside the treatment room. In one exemplary implementation, automatized exchange of field-specific elements are done by utilizing (universal) industrial robots, to avoid any handling of these elements by clinical personnel. Figure 19 is a block diagram of an exemplary system 1900 in accordance with one embodiment. System 1900 includes treatment and configuration control component 1910, local field fabrication system 1921, local fabricated part station 1922, remote fabricated part station 1930, modulation scanning component 1940, and robotic components 1951, and 1952. In one embodiment, treatment and configuration control component 1910 is similar to treatment and configuration control component 109 (Figure 1). In one exemplary implementation, modulation scanning component 1940 is similar to modulation scanning component 107. Local field fabrication system 1921 can be similar to modulation component fabrication system 1680. Local fabricated part station 1922 and remote fabricated part station 1930 can hold modulation components/parts. Robotic components 1951, and 1952 can automatically move items (e.g., modulation components/parts, etc.) between local fabricated part station 1922 and remote fabricated part station 1930 and modulation scanning component 1940.

It is appreciated that aspects of the presented modulation component systems and methods can include improved radiation treatment system performance and radiation treatment process results. A very important aspect of medical procedures involving radiation is to reliably deliver appropriate radiation treatment to desired tissue targets (e.g., tumors, etc.) while avoiding other tissue (e.g., organs at risk, etc.). Aspects of proper radiation delivery (e.g., proper doses, dose rates, depths, etc.) are typically dependent on accurate and reliable particle beam modulation. While there are similarities in human anatomy, there are usually variations in each patient (e.g., as no two are exactly alike, etc.) making realization of precise and accurate radiation delivery difficult. The presented modulation component systems' and methods' ability to be customized for each patient significantly overcomes traditional problems associated with differences in patients. The presented modulation component systems can enable more accurate and precise radiation delivery results on an individual basis than conventional approaches.

The presented modulation component systems and methods can offer significant improvements in areas conventionally limited by time constraints. Reducing the amount of time consumed by pauses and stops traditionally associated with particle beam energy changes enables realization of high dose rate approaches (e.g., FLASH protocols, etc.) that were not possible/practical with conventional systems and methods. In addition, automatic performance of certain treatment plan and modulation component development and adjustment increases radiation treatment system performance and radiation treatment process results. In one embodiment, automatic performance of certain treatment plan and modulation component development and adjustment in accordance with presented novel algorithms make improved radiation treatment possible/practical, unlike traditional approaches. The automatic performance of certain treatment plan and modulation component development and adjustment is implemented utilizing computer processing that can meet crucial timing/performance characteristics that cannot be realized otherwise on a practical/actual level. Many aspects of radiation treatment have practical/requisite timing constraints (e.g., patients can only remain in the same position for limited time, tumors typically change or grow over time, etc.). In one exemplary implementation, the amount of information processing and intelligent development cannot be performed by human minds in a practical/requisite manner that meets timing constraints, preventing traditional systems and methods from realizing improvement in performance and results. In one embodiment, the modulation component systems and methods also allow for rapid adjustment response (e.g., overcome unforeseen conditions, optimization, fix quality problems, etc.) improvements in both the treatment plan and modulation component implementation that are not realizable/practical in traditional approaches.

While most of the description is explained with an emphasis on medical radiation therapy applications, it is appreciated the present systems and methods are readily implemented and utilized in a variety of other applications. In one embodiment, the systems and methods are utilized for other types of RT treatments besides FLASH. The scanning and particle beam spread control is utilized in conjunction with an X-ray target utilized in Bremsstrahlung creation of X-rays. In one embodiment, the described particle beam distribution and spread adjustment control are utilized in industrial products/applications.

Some portions of the detailed descriptions are presented in terms of procedures, logic blocks, processing, and other symbolic representations of operations on data bits within a computer memory. These descriptions and representations are the means used by those skilled in the data processing arts to most effectively convey the substance of their work to others skilled in the art. In the present application, a procedure, logic block, process, or the like, is conceived to be a self-consistent sequence of steps or instructions leading to a desired result. The steps are those utilizing physical manipulations of physical quantities. Usually, although not necessarily, these quantities take the form of electrical or magnetic signals capable of being stored, transferred, combined, compared, and otherwise manipulated in a computer system. Portions of the detailed description are presented and discussed in terms of methods. Although steps and sequencing thereof are disclosed in figures herein describing the operations of those methods, such steps and sequencing are examples only. Embodiments are well suited to performing various other steps or variations of the steps recited in the flowcharts of the figures herein, and in a sequence other than that depicted and described herein.

It should be borne in mind, however, that all of these and similar terms are to be associated with the appropriate physical quantities and are merely convenient labels applied to these quantities. Unless specifically stated otherwise as apparent from the following discussions, it is appreciated that throughout the present disclosure, discussions utilizing terms such as "determining," "accessing," "generating," developing," "performing," planning," "representing," "applying," "indicating," "storing," "using," "adjusting," "including," "computing," "displaying," "associating," "rendering," "determining," or the like, refer to actions and processes of a computer system or similar electronic computing device or processor. The computer system or similar electronic computing device manipulates and transforms data represented as physical (electronic) quantities within the computer system memories, registers or other such information storage, transmission or display devices. Terms such as "dose" or "dose rate" or "fluence" generally refer to a dose value or dose rate value or fluence value, respectively; the use of such terms will be clear from the context of the surrounding discussion.

Although the subject matter has been described in language specific to structural features and methodological acts, it is to be understood that the subject matter defined in the appended claims is not necessarily limited to the specific features or acts described above. Rather, the specific features and acts described above are disclosed as example forms of implementing the claims.

## Claims

1. A system comprising:
a particle generation component configured to generate a particle beam;
a modulation scanning component configured to control movement of the particle beam in a scan pattern and modulation of the particle beam resulting in a modulated treatment particle beam; and
a treatment and configuration control component configured to direct configuration of the modulation scanning component and configured to direct delivery of the treatment particle beam, wherein the configuration of the modulation scanning component and delivery of the treatment particle beam are based upon a treatment plan, wherein the particle generation component is configured to generate a treatment particle beam at a same energy level for a first portion of the scan pattern and a second portion of the scan pattern, and the modulation scanning component modulation of the treatment particle beam is configured to be different for the first portion of the scan pattern than the second portion of the scan pattern.

2. The system of claim 1, wherein the modulation scanning component is partitioned into a plurality of pin cells in which a first one of the plurality of pin cells and a second one of the plurality of pin cells have different configurations that are configured to result in different modulation of the treatment particle beam.

3. The system of Claim 1 or Claim 2, wherein a range of the treatment particle beam is configured to be different for the first portion of the scan pattern and the second portion of the scan pattern.

4. The system of any preceding Claim configured so that an adjustment of the modulation scanning component of a treatment particle beam includes shifting a deposition depth of the treatment particle beam to a lower depth.

5. The system of any preceding Claim configured so that an adjustment of the modulation scanning component of a treatment particle beam includes generating a determined dose profile from a largest penetration depth to a smallest penetration depth;
and preferably configured so that:
the determined dose profile is homogenous from the largest penetration depth to the smallest penetration depth.

6. The system of any preceding Claim, configured so that an adjustment of the modulation scanning component of the treatment particle beam applies fields with multiple Iso-Energy-Slices (IES) and the treatment particle beam is at the same energy level for the first portion of the scan pattern and the second portion of the scan pattern;
and/or configured so that:
treatment fields are irradiated as single Iso-Energy-Slice (IES) fields by the treatment particle beam.

7. The system of any preceding Claim, configured so that the modulation scanning component includes homogenous and field individual modulation components that allow a conformal irradiation using the treatment particle beam at the same energy level.

8. The system of any preceding Claim, wherein the treatment and configuration control component is configured to develop an optimized version of the treatment plan, and the system is configured so that adjustment of the treatment particle beam by the modulation scanning component in accordance with information from the treatment and configuration control component optimizes radiation treatment in a target tissue.

9. The system of any preceding Claim, wherein the treatment and configuration control component is configured for adjustment of the treatment particle beam and radiation in optimization of the treatment plan;
and/or:
the system is configured so that an adjustment of the treatment particle beam by the modulation scanning component optimizes on a scan point location and depth granularity basis.

10. The system of any preceding Claim, configured so that an adjustment of the treatment particle beam by the modulation scanning component optimizes dose distribution;
and/or the system is configured so that:
an adjustment of the treatment particle beam by the modulation scanning component optimizes dose rate.

11. A method comprising:
performing a treatment plan creation process in which a treatment plan is created;
performing a modulation component configuration process, wherein a modulation component is configured based on the treatment plan; and
performing a quality assurance process, including a quality assurance process on the modulation component.

12. The method of Claim 11 wherein the treatment plan creation process includes planning and performing a CT scan of a patient;
and/or:
wherein the treatment plan creation process includes determining a dose prescription and developing a remainder of the treatment plan to achieve the dose prescription.

13. The method of Claim 11 or Claim 12 wherein a creation process of the treatment plan includes utilizing laterally distributed scan spot positions corresponding to modulator pin cells of a modulator component, wherein the modulator pin cells are configured to receive a particle beam at a similar energy level across a plurality of the modulator pin cells,
and preferably:
wherein the similar energy level is a highest available energy of a treatment system.

14. The method of any of Claim 11 to Claim 13 wherein the treatment plan creation process includes laterally distributed scan spot positions which are arranged in a scan pattern.

15. The method of any of Claim 11 to Claim 14 wherein the quality assurance process includes performing a CT scan of the created modulation component and using the CT scan to verify the modulation component;
and preferably:
wherein results of the CT scan are compared to desired values from a simulated CT scan.
